# EUROPÄISCHE PATENTSCHRIFT

(11) **EP 1 471 948 B1**
(45) Veröffentlichungstag und Bekanntmachung des Hinweises auf die Patenterteilung: **31.08.2005**
(21) Anmeldenummer: 03702502.0
(22) Anmeldetag: 23.01.2003
(51) Int. Cl.: A61L 9/012, A61L 9/014, A61L 9/04, B01D 53/02, A61L 9/16

(54) **ENTFERNEN BZW. REDUZIEREN VON BELRIECHENDEN SUBSTANZ EN IN DER LUFT**
REMOVAL OR REDUCTION OF MALODOROUS SUBSTANCES IN THE AIR
ELIMINATION COMPLETE OU PARTIELLE DE SUBSTANCES MALODORANTES PRESENTES DANS L'AIR

(30) Priorität: 29.01.2002 DE 10203339; 14.03.2002 DE 10211165
(43) Veröffentlichungstag der Anmeldung: 03.11.2004
(73) Patentinhaber: Air & D- Sarl, 67190 Rosheim (FR)
(72) Erfinder: WUEST, Robert, F-67190 Rosheim (FR)
(74) Vertreter: Patentanwälte Zellentin & Partner
(86) Internationale Anmeldenummer: PCT/EP2003/000641
(87) Internationale Veröffentlichungsnummer: WO 2003/063920

(56) Entgegenhaltungen:
- WO-A-01/78794
- WO-A-98/07454
- US-A- 4 339 550
- US-A- 5 230 958
- US-A- 5 569 683

## Beschreibung

Die Erfindung betrifft die Verwendung eines Adsorbens, welches eine offenzellige Schwammstruktur aufweist, zum Entfernen bzw. Reduzieren von übelriechenden Substanzen in der Luft.

Zum Entfernen bzw. Reduzieren von übelriechenden Substanzen in der Luft durch Gaswäsche werden in der Praxis verschiedenartige Methoden angewandt. Einige davon nutzen die Fähigkeit von Festsubstanzen mit großer Oberfläche, gasförmige Substanzen zu adsorbieren und damit über einen längeren Zeitraum hinweg zu binden. So werden z. B. mineralische Trägerrnaterialien, Papiervliese und Aktivkohle als Filtereinsätze zum Entfernen von flüchtigen Verunreinigungen aus Luft- und Abluftströmen verwendet.

Ein anderes Prinzip besteht darin, dass man Pulver, saugfähige Papiere, aufnahmefähige Polymere oder hochporöse Tongranulate mit aktiven Agentien, die mit übelriechenden Substanzen reagieren oder diese maskieren können, imprägniert. Sie geben diese aktiven Agentien langsam wieder ab und können dadurch ihre Wirkung entfalten, wenn sie von verunreinigter Luft durch- oder überströmt werden.

WO-A-01/78794 offenbart ein wasserfreies Gelelement, welches durch Vernetzung von maleinisiertem Polybutadien mit einem Amin in Gegenwart eines nichtwässrigen Parfüms und einem metallfreien Farbstoff zur Desodorierung von Luft erzeugt wird.

Der Erfindung lag nun die Aufgabe zugrunde, ein einfach herstellbares, hochwirksames Adsorbens bereitzustellen, welches in der Lage ist, sowohl übelriechende Substanzen direkt zu binden als auch aktive Agentien zu adsorbieren und desorbieren, die mit den übelriechenden Substanzen in der Luft reagieren bzw. diese maskieren können.

Diese Aufgabe wird durch die Verwendung eines vernetzten, hydrophile Gruppen enthaltenden maleinisierten oder epoxidierten Polymeren gelöst, welches mit einer Flüssigkeit angequollen ist und mit dieser zusammen eine offenzellige Schwammstruktur ausbildet, wobei die schwammartige Masse Wasser und gegebenenfalls einen Alkohol enthält, zum Entfernen bzw. Reduzieren von übelriechenden Substanzen in der Luft.

Die vernetzten maleinisierten bzw. epoxidierten Polymeren sind Umsetzungsprodukte eines hydrophile Gruppen tragenden Polymeren mit einem Vemetzer. Geeignete hydrophile Gruppen tragende Polymere sind maleinisierte oder epoxidierte Polymere, z. B. Umsetzungsprodukte von Polydienen mit einem Molekulargewicht zwischen 400 und 10 000, beispielsweise Polybutadien, Polydecadien und ungesättigte Fettsäuren, wie Soyabohnenöl, mit Maleinsäureanhydrid, ferner Copolymere von Olefinen, wie Ethylen, mit Maleinsäureanhydrid, sowie epoxidiertes Polybutadien. Auch Kohlehydrate, wie vernetzte Fructose und Stärke, können als Polymer eingesetzt werden. Bevorzugte Vemetzer sind Polyamine mit 2 oder 3 Aminogruppen, insbesondere Polyoxyalkylenpolyamine, wie Polyoxypropylendiamin und Polyoxypropylentriamin. Neben Polyaminen können auch Harnstoff, Polyethylenimin, Epichlorhydrin, Triethylenglykol, sowie Zinkoxid, Zinkacetat und Calciumhydroxid als Vernetzer wirken, wobei letztere wahrscheinlich mit zwei Maleinsäureanhydrid-Gruppen einen stabilen Komplex bilden. Die Herstellung der bevorzugten vernetzten, hydrophile Gruppen tragenden Polymeren erfolgt durch Kondensation des maleinisierten bzw. epoxidierten Polymeren mit dem Polyamin, vorzugsweise in alkoholischer Lösung, insbesondere in Dipropylenglykol, bei erhöhter Temperatur, vorzugsweise zwischen 40° und 60° C. Auch hier ist das bevorzugte Molverhältnis Polymer zu Vemetzer 20 : 1 bis 1 : 1, insbesondere 8 : 1 bis 1 : 1. Bei einem niedrigen Molverhältnis nahe 1 : 1 erhält man eine starke Vernetzung, was relativ zähe und kompakte schwammartige Massen ergibt. Die Kondensation wird in Gegenwart von Zusatzstoffen, wie quellend wirkenden und oberflächenaktiven Flüssigkeiten, insbesondere von aktiven Agentien durchgeführt. Als hydrophile Gruppen wirken insbesondere die von den Polyoxyalkylenpolyaminen stammenden -CRH-O- Gruppen, daneben die Maleinsäureanhydrid- und Carboxyl-Gruppen bzw. die -NR-CO- Gruppen des vernetzten maleinisierten Polymeren, sowie die Epoxidgruppen bzw. -COH-CNR- Gruppen des vernetzten epoxidierten Polymeren.

Das vernetzte Polymere bildet zusammen mit den oberflächenaktiven Flüssigkeiten eine offenzellige Schwammstruktur, die in der Lage ist, weitere Flüssigkeiten und insbesondere Gase zu adsorbieren. Sie kann dabei mehr als das Vierfache, in manchen Fällen sogar mehr als das Zehnfache ihres Gewichts an flüchtigen Fremdsubstanzen aufnehmen. Das vernetzte Polymere weist ein räumliches Netzwerk mit Poren auf, in dem die flüchtigen Fremdsubstanzen aufgesaugt und adsorbiert werden, so dass das Polymere wie ein Schwamm anquillt. Im angequollenen Zustand besteht das dreidimensionale Netzwerk aus Elementarzellen, die im Mittel ein Volumen von 1 bis 1000 nm³, vorzugsweise von 3 bis 200 nm³ aufweisen.

Die erfindungsgemäßen vernetzten Polymeren werden direkt oder indirekt zum Entfernen bzw. Reduzieren von übelriechenden Substanzen in der Luft, z. B. von Ammoniak, Schwefelwasserstoff, Chlor, Phenolen, organischen Amino-, Schwefel- und Halogenverbindungen, sowie von flüchtigen Kohlenwasserstoffen verwandt.

Bei einer ersten Ausführungsform der Erfindung wird das vernetzte Polymere direkt zum Adsorbieren und damit zum Entfernen der übelriechenden Substanzen aus der Luft eingesetzt. Dabei sollte das Polymere schon mit einer Flüssigkeit zu der schwammartigen Masse angequollen sein, außer mit Wasser z.B. mit Paraffinöl, einem Alkohol, einem Glykol oder einem oberflächenaktiven Mittel, z. B. einem Polyalkylenglykol, vorzugsweise Dipropylenglykol in Mengen von 5 bis 1000 Gew.-%, insbesondere von 50 bis 500 Gew.-%, bezogen auf das Polymere. Günstig ist auch der Zusatz von fein verteilten Feststoffen, die die Adsorption unterstützen, z. B. von Silicagel, Zinkoxid oder Silikaten in Mengen von 0,5 bis 5 Gew.-%, bezogen auf das Polymere, sowie von Zucker und/oder organischen Bromverbindungen als Flammschutzmittel.
Bevorzugt ist auch der Zusatz von Feststoffen oder Flüssigkeiten in Mengen von 2 bis 400 Gew.-%, bezogen auf das Polymere, die in der Lage sind, mit übelriechenden Substanzen zu reagieren und sie damit chemisch zu binden, z. B. von Ascorbinsäure, Gallussäure, Zinkgluconat, Calciumlactat, Benzylsalicylat, cis- 3- Hexensalicylat, Butylhydroxytoluol und Tocopherol.

Die genannten Feststoffe und Flüssigkeiten können entweder bereits bei der Herstellung der Polymeren zugesetzt werden oder anschließend mit diesen vermischt werden.

Zum Entfernen der übelriechenden Substanzen aus der Luft kann man diese, z. B. in einem Abluftrohr oder einem Kamin, über eine Polymerschicht oder über Polymerpartikel streichen lassen. Aufgrund ihrer schwammartigen Struktur können sie auch direkt als Filter wirken. Es ist auch möglich, die Polymeren als Emulsion in einer Flüssigkeit verteilt in Form feiner Tröpfchen mit einem Durchmesser von 0,5 bis 500 µm auf eine Oberfläche sprühen, an der die verunreinigte Luft vorbeiströmt.
Die vernetzten Polymeren wirken aufgrund ihrer netzartigen Struktur wie ein Schwamm, der die übelriechenden Substanzen aufsaugt und bindet. Nach einer gewissen Zeit, deren Dauer durch einfache Versuche ermittelt werden kann, sind die Polymeren gesättigt; sie können dann z. B. in einem Säurebad regeneriert werden.

Bei einer zweiten, bevorzugten Ausführungsform der Erfindung wird das vernetzte Polymere mit einem aktiven Agens beladen und bildet mit diesem eine schwammartige Masse. Das aktive Agens wird daraus langsam und gleichmäßig freigesetzt und kann dann mit den übelriechenden Substanzen reagieren, diese reduzieren bzw. maskieren.

Die aktiven Agentien sind meist flüssige Aldehyde, Alkohole, Ketone, Terpene oder Ester, die mit den übelriechenden Substanzen reagieren können oder als Parfüme üble Gerüche überdecken und maskieren. Beispiele für geeignete Flüssigkeiten sind Vanillin, Eugenol, Thymol, Geraniol, Citronellol, Eucalyptol, Jasmonal H, Linanol, Menthol, Cumarin, Citral, Alpha-Pinen, die Aldehyde C7 bis C12, Nerylacetat, Linalylacetat, Butylhydroxytoluol und Salicylsäurebenzylester, ferner natürliche ölige Essenzen, wie Harzöl, sowie Mischungen dieser Flüssigkeiten.

Vorzugsweise werden die aktiven Agentien bei der Herstellung der vernetzten Polymeren durch Polymerisation oder Kondensation den Ausgangsmaterialien zugesetzt. Man kann auch das vernetzte Polymere mit den aktiven Agentien tränken und damit anquellen. Das aktive Agens sollte in der schwammartigen Masse in Mengen von 10 bis 90 Gew.-%, insbesondere von 40 bis 80 Gew.-% enthalten sein.
Auch hier kann die schwammartige Masse Zusatzstoffe enthalten, außer Wasser z.B. Flammschutzmittel, wie Zucker, Azodicarbonamid und Bromverbindungen, ferner Sägemehl oder andere Pulver zur Verhinderung des Verbackens, Lösungsmittel, z. B. Alkohole, sowie Sublimationshilfsmittel, welche die Freisetzung des aktiven Agens beschleunigen.

Eine typische Zusammensetzung der Ausgangsmaterialien bei der Herstellung der schwammartigen Masse sieht folgendermaßen aus:

| | |
|---|---|
| maleinisierte Polymere | 14 - 30 Gew.-% |
| Vernetzer | 0,2 - 5 Gew.-% |
| Wasser | bis 10 Gew.-% |
| Flammschutzmittel | 1 - 20 Gew.-% |
| Aktives Agens | Rest |

Wesentlich ist, dass das aktive Agens mit dem zur Anwendung kommenden vernetzten Polymeren so abgestimmt ist, dass es nur sehr langsam und während des Wirkungszeitraums gleichmäßig aus der schwammartigen Masse freigesetzt wird und seine Wirkung mindestens drei Tage, vorzugsweise mindestens eine Woche und insbesondere einen bis sechs Monate lang beibehält. Das freigesetzte aktive Agens kann mit den übelriechenden Substanzen in der Gasphase reagieren und dabei die übelriechenden Substanzen aus der Luft entfernen bzw. reduzieren. Neben eigentlichen chemischen Reaktionen, z. B. zwischen Schwefelwasserstoff oder Ammoniak und Aldehyden kann es dabei auch zu Bindungen durch elektrostatische oder van der Waals'sche Kräfte kommen, wodurch die Geruchswahrnehmbarkeit zumindest herabgesetzt wird. Weiterhin können die aktiven Agentien auch die üblen Gerüche maskieren und/oder als spezielle Parfümkomposition den üblen Geruch überdecken und eine Beduftung erzielen.
Je nach Anwendungszweck liegt die schwammartige Masse, die das aktive Agens enthält, als Krümel, Späne, Granulat, Kugeln, Würfel, Streifen oder Platten vor.

Natürlich können die beiden Ausführungsformen auch miteinander kombiniert werden.

Folgende praktische Anwendungen sind möglich:

Bei Deponien, auf denen übelriechender Müll bzw. Schlamm mit Erde abgedeckt ist. Die schwammartige Masse wird z. B. als Späne mit dem Müll vermischt, wobei 10 bis 100 g des aktiven Agens pro m² genügen.

In Tierzuchtanlagen, Ställen, Güllebehältern oder zoologischen Gärten, wobei die schwammartige Masse in Form von Blöcken, Platten, Streifen oder Krümel auf Gitter oder Netze aufgelegt wird. Diese können direkt an der Decke aufgehängt werden; vorzugsweise werden aber mehrere Gitter übereinander oder nebeneinander auf einem Gestell angeordnet. Dieses kann in einem Ventilationskasten eingebaut werden, der im Raum oder in Eingangsluftschächten angebracht wird. In letzterem bewirkt die angesaugte Frischluft die Verdunstung der aktiven Agentien.

In geschlossenen Räumen, z. B. in Zimmern, Aufenthaltsräumen, Hallen, Küchen und Toiletten von Krankenhäusern, Altersheimen und insbesondere von Hotels und Restaurants. Dabei kann die schwammartige Masse in Form von Krümeln, Streifen, Platten oder Blöcken direkt in Klimaschächte eingebracht werden; vorzugsweise werden sie aber auf Gitter oder Netze aufgelegt. Diese können wiederum übereinander auf einem Gestell in einem Ventilationskasten eingebracht werden.

In öffentlichen Verkehrsmitteln, wie Eisenbahn-, Straßenbahn-, Untergrundbahn-Waggons, Bussen, sowie See- und Luftfahrzeugen. Auch hier kann die schwammartige Masse in Form von Krümeln, Streifen, Platten oder Blöcken auf Netze oder Gitter aufgelegt und diese übereinander in einem Gestell angeordnet werden, das in den Luftschacht des Verkehrsmittels eingebracht wird.

In Untergrundbahnstationen, wo die schwammartige Masse z. B. an der Kante zwischen Bahnsteig und dem Schienenbereich angebracht werden kann.

In Industrieanlagen, wobei die schwammartige Masse in Abluftkaminen bzw. in Lufteingangsöffnungen oder in die Einblasvorrichtung für Prozessluft angebracht sind. Wo mit starker Verschmutzung zu rechnen ist (z. B. bei Vulkanisationsanlagen) oder wo Abluft mit hoher Temperatur eintritt, welche die Polymermatrix schädigen könnte, kann das aktive Agens in den Raum hinein eingeblasen oder aus einem seitlich angebrachten Einsatz durch Unterdruck eingesaugt werden, wo es sich mit der Abluft vermischen und mit den übelriechenden Substanzen reagieren kann.

Auch dort, wo eine Zwangslüftung nicht erfolgt, kann die schwammartige Masse in separat montierbare, speziell gefertigte Ventilatoren eingesetzt werden, die dann für eine gleichmäßige Verteilung der austretenden Agentien sorgen.

### Beispiel 1

10 g maleinisiertes Polybutadien (Umsetzungsprodukt aus flüssigem Polybutadien mit Maleinsäureanhydrid - LITHENE der Fa. Revertex) wurden bei 50° C mit 40 g Dipropylenglykol vermischt (Mischung A). 3 g Polyoxypropylendiamin (MG 400) wurden bei Raumtemperatur mit 45 g Dipropylenglykol vermischt (Mischung B). Die Mischungen A und B wurden in einem flachen Gefäß bei 40° C 15 min. lang zusammen mit 1 g Wasser und 1 g Surfectant LRI der Fa. Wacker (eine Mischung aus 26% Polypropylenglykol, 40%Polyethylenglykol, 26%Butylethylglykol und 8% hydriertes Castoröl) verrührt.

Das erhaltene, feine Poren enthaltende, schwammartige Masse wurde direkt als Filter zum Entfernen von übelriechenden Substanzen in der Luft verwendet.

### Beispiel 2

In einem 2,5 I-Reaktor wurden 100 g Stärke mit einem MG von 10.000 in 300 ml Wasser gelöst. 1 g Natriumborhydrid als Katalysator, 100 g eines Aldehyds als aktivem Agens und 20 g Surfectant LRI wurden eingerührt. Nach 2 Stunden Rühren mit 200 rpm bei Raumtemperatur wurden 30 ml Epichlorhydrin als Vemetzer unter Rühren mit 100 rpm zugefügt. Die Mischung gelierte und ergab eine schwammartige Masse. Diese wurde in Krümel zerkleinert und mit 400 mg Amyloglucosidase vermischt, welche die Stärke enzymatisch abbaut und damit die Zersetzung der schwammartigen Masse und Freisetzung des aktiven Agens beschleunigt. Die Krümel wurden mit Erde vermischt und auf eine Mülldeponie aufgetragen.

## Patentansprüche

1. Verwendung eines vernetzten, hydrophile Gruppen enthaltenden maleinisierten oder epoxidierten Polymeren, welches mit einer Flüssigkeit angequollen ist und mit dieser zusammen eine offenzellige Schwammstruktur bildet, zum Entfernen bzw. Reduzieren oder zum Maskieren von übelriechenden Substanzen in der Luft, **dadurch gekennzeichnet, dass** die schwammartige Masse Wasser und gegebenenfalls einen Alkohol enthält.

2. Verwendung nach Anspruch 1, **dadurch gekennzeichnet, dass** die schwammartige Masse bis 10 Gew.% Wasser enthält.

3. Verwendung nach Anspruch 1, **dadurch gekennzeichnet, dass** die schwammartige Masse als Alkohol ein Polyalkylenglykol, vorzugsweise Dipropylenglykol enthält.enthält.

4. Verwendung nach Anspruch 1, **dadurch gekennzeichnet, dass** das vernetzte Polymere ein Umsetzungsprodukt aus einem maleinisierten oder epoxidierten Polymeren mit einem Molekulargewicht von mehr als 400 und einem Polyamin als Vemetzer ist.

5. Verwendung nach Anspruch 4, **dadurch gekennzeichnet, dass** das Polymere ein Polydiolefin ist, vorzugsweise Polybutadien oder Polydecadien.

6. Verwendung der vernetzten Polymeren nach Anspruch 1 zum Reduzieren von übelriechenden Substanzen in der Luft durch chemische Reaktion von flüssigen aktiven Agentien mit den Substanzen oder durch Maskieren der Substanzen mit den aktiven Agentien, **dadurch gekennzeichnet, dass** die aktiven Agentien an dem vernetzten Polymeren adsorbiert sind und mit diesem zusammen eine schwammartige Masse bilden, und dass die aktiven Agentien aus der schwammartigen Masse langsam und gleichmäßig freigesetzt werden und verdunsten und erst dann mit den übelriechenden Substanzen reagieren bzw. diese maskieren.

7. Verwendung nach Anspruch 6, **dadurch gekennzeichnet, dass** die aktiven Agentien in der schwammartigen Masse in einer Menge von 10 bis 90 Gew.-% enthalten sind.

8. Verwendung nach Anspruch 6, **dadurch gekennzeichnet, dass** die aktiven Agentien Aldehyde, Ketone, Alkohole, Ester, Terpene oder natürliche ölige Essenzen sind.

9. Verwendung der vernetzten Polymeren nach Anspruch 1 zum Entfernen von übelriechenden Substanzen aus der Luft, **dadurch gekennzeichnet, dass** die Substanzen an einer schwammartigen Masse aus dem vernetzten Polymeren und quellenden oder oberflächenaktiven Flüssigkeiten adsorbiert werden.

10. Verwendung der schwammartigen Masse nach Anspruch 1 zum Desodorieren von Tierzuchtanlagen, Ställen und Güllebehältern, insbesondere in Schweinemastanlagen.

11. Verwendung der schwammartigen Masse nach Anspruch 1 zum Desodorieren von Gebäuderäumen, insbesondere in Hotels und Restaurants.

12. Verwendung der schwammartigen Masse nach Anspruch 1 zum Desodorieren von öffentlichen Verkehrsmitteln.

## Claims

1. The use of a crosslinked maleinized or epoxidized polymer containing hydrophilic groups which is swollen with a liquid, forming with the liquid an open-cell sponge structure, for removing, reducing or masking malodorous substances in the air, **characterized in that** the open-cell sponge structure contains water, together with or without an alcohol.

2. Use as claimed in claim 1, **characterized in that** the open-cell sponge structure contains up to 10% by weight of water.

3. Use as claimed in claim 1, **characterized in that** the open-cell sponge structure contains as an alcohol a polypropylene glycol, preferably dipropylene glycol.

4. Use as claimed in claim 1, **characterized in that** the crosslinked polymer is a reaction product of a maleinized or epoxidized polymer having a molecular weight of more than 400 and a polyamine as crosslinking agent.

5. Use as claimed in claim 4, **characterized in that** the polymer is a polydiolefin, preferably polybutadiene or polydecadiene.

6. Use of crosslinked polymers as claimed in claim 1 for reducing malodorous substances in the air by means of a chemical reaction of liquid active agents with the substances or by masking the substance with the active agents, **characterized in that** the active agents are adsorbed in the crosslinked polymer, forming with it a spongelike composition, and **in that** the active agents are slowly and uniformly released from the spongelike composition, thereby volatilizing and only then react with the malodorous substances or mask these.

7. Use as claimed in claim 6, **characterized in that** the active agents in the spongelike composition are contained in an amount of 10 to 90% by weight.

8. Use as claimed in claim 6, **characterized in that** the active agents are aldehydes, ketones, alcohols, esters, terpenes or natural oily essences.

9. Use of the crosslinked polymers as claimed in claim 1 for removing malodorous substances from the air, **characterized in that** the substances are adsorbed in a spongelike composition of the crosslinked polymer and swelling or surface-active liquids.

10. Use of a spongelike composition as claimed in claim 1 for deodorizing animal breeding facilities, stables and liquid manure containers, especially in pig breeding facilities.

11. Use of the spongelike composition as claimed in claim 1 for deodorizing rooms in buildings, especially in hotels and restaurants.

12. Use of the spongelike composition as claimed in claim 1 for deodorizing public means of transport.

## Revendications

1. L'utilisation d'un polymère maléinisé ou époxydé, étant réticulé et contenant des groupes hydrophiles qui est gonflé avec un liquide et qui constitue avec ce dernier une structure spongieuse à cellules ouvertes pour enlever ou bien réduire ou masquer des substances malodorantes de l'air, **caractérisé par le fait que** la masse spongieuse contient de l'eau et, le cas échéant, un alcool.

2. L'utilisation selon la revendication **1, caractérisé par le fait que** la masse spongieuse contient jusqu'à 10% en poids en eau.

3. L'utilisation selon la revendication **1, caractérisé par le fait que** la masse spongieuse contient comme alcool un polyalkylène-glycol, de préférence un dipropylène-glycol.

4. L'utilisation selon la revendication 1, **caractérisé par le fait que** le polymère réticulé est un produit de réaction d'un polymère maléinisé ou époxydé avec un poids moléculaire de plus de 400 avec un polyamine en tant que réticulant.

5. L'utilisation selon la revendication 4, **caractérisé par le fait que** le polymère est un polydioléfine, de préférence un polybutadiène ou un polydécadiène.

6. L'utilisation des polymères réticulés selon la revendication 1 pour la réduction des substances malodorantes de l'air par des réactions chimiques des agents actifs liquides avec ces substances ou par le masquage des substances avec les agents actifs, **caractérisé par le fait que** les agents actifs sont adsorbé dans le polymère réticulé et forment avec celui-ci une masse spongieuse et que les agents actifs se libèrent de la masse spongieuse de manière lente et régulière et s'évaporent et ce n'est qu'à ce moment-là qu'ils réagissent avec ou bien masquent les substances malodorantes.

7. L'utilisation selon la revendication 6, **caractérisé par le fait que** les agents actifs contenus dans la masse spongieuse selon une proportion de 10 à 90 % en poids.

8. L'utilisation selon la revendication 6, **caractérisé par le fait que** les agents actifs sont des aldéhydes, cétones, alcools, esters, terpènes ou autres essences végétales.

9. L'utilisation des polymères réticulés selon la revendication 1 pour enlever des substances malodorantes de l'air, **caractérisé par le fait que** les substances sont adsorbées dans une masse spongieuse constituée d'un polymère réticulé et par des liquides gonflants ou de surface active.

10. L'utilisation de la masse spongieuse selon la revendication 1 pour la désodorisation des installations d'élevage, des étables et des fosses à lisier, en particulier pour les installations d'engraissement de porcs.

11. L'utilisation de la masse spongieuse selon la revendication 1 pour la désodorisation des pièces, en particulier des hôtels et restaurants.

12. L'utilisation de la masse spongieuse selon la revendication 1 pour la désodorisation des transports en commun.
